# EUROPÄISCHE PATENTSCHRIFT

(11) **EP 2 875 867 B1**
(45) Veröffentlichungstag und Bekanntmachung des Hinweises auf die Patenterteilung: **01.06.2016**
(21) Anmeldenummer: 13194408.4
(22) Anmeldetag: 26.11.2013
(51) Int. Cl.: B01L 3/08, C12M 1/24

(54) **Kulturgefäß / Schraubdeckelgefäß**
Culture vessel/vessel with screw-on lid
Récipient de culture / récipient à couvercle à visser

(43) Veröffentlichungstag der Anmeldung: 27.05.2015
(73) Patentinhaber: EPPENDORF AG, 22339 Hamburg (DE)
(72) Erfinder: Seippel, Martin, 22949 Ammersbek (DE)
(74) Vertreter: Hauck Patentanwaltspartnerschaft mbB

(56) Entgegenhaltungen:
- WO-A1-97/31833
- WO-A1-2007/071287
- US-A- 5 578 491

## Beschreibung

Die Erfindung bezieht sich auf ein Schraubdeckelgefäß für den Laboreinsatz. Insbesondere bezieht sich die Erfindung auf ein Schraubdeckelgefäß für den Einsatz im biologischen, mikrobiologischen, medizinischen, chemischen oder pharmazeutischen Labor.

Das Schraubdeckelgefäß ist beispielsweise eine Kulturflasche oder ein anderes Kulturgefäß zum Kultivieren von Zellen, Geweben oder Mikroorganismen oder eine Laborflasche oder anderes Laborgefäß zum Aufbewahren von Flüssigkeiten und anderen Medien.

Kulturgefäße werden im Labor zur Anzucht und Gewinnung von Zellen, Geweben oder Mikroorganismen verwendet. Sie haben in einem Gefäßkörper eine Kulturkammer, die durch eine Gefäßöffnung zugänglich ist. Zum abdichtenden Verschließen der Gefäßöffnung weisen sie eine Schraubkappe auf. Für eine Begasung des Mediums in der Kulturkammer z.B. durch CO₂ im Inkubator kann die Schraubkappe etwas losgeschraubt werden. Hierbei kann nicht sichergestellt werden, dass ein ausreichender, reproduzierbarer Belüftungsquerschnitt erreicht wird. Zudem kann sich die Schraubkappe aufgrund von Vibrationen des Inkubators vollständig lösen und herunterfallen.

Ferner sind Kulturflaschen bekannt, die in einer Schraubkappe einen Membranfilter aufweisen, um einen Gasaustausch zwischen dem Flascheninneren und der Umgebung zu ermöglichen, wenn die Schraubkappe zugeschraubt ist. Diese Kulturflaschen haben den Nachteil, dass Flüssigkeit austreten kann, insbesondere wenn die Zellkulturflasche gekippt wird, sodass Flüssigkeit an der Membran oder dem Filter ansteht.

Bekannt sind auch schon Zellkulturflaschen mit einer Schraubkappe, die in einer definierten Belüftungsstellung einrastet, in der die Schraubkappe noch nicht abdichtet. Durch weiteres Zuschrauben der Schraubkappe über die Belüftungsstellung hinaus ist die Schraubkappe in die Dichtstellung bringbar, in der sie die Zellkulturflasche gas- und flüssigkeitsdicht abdichtet. Für die Verrastung in der Belüftungsstellung weist die Zellkulturflasche mehrere Nocken auf dem Flaschenhals und korrespondierende axial gerichtete Nuten am Innenumfang der Schraubkappe auf. Beim Zuschrauben des Deckels greifen die Nocken nach Überwindung eines leichten Widerstandes in die Nuten ein, bevor die Schraubkappe die Gefäßöffnung verschließt. In dieser Raststellung ist das Flascheninnere belüftet. Beim weiteren Zudrehen der Schraubkappe, wiederum gegen leichten Widerstand, verlassen die Nocken die Nuten und dichtet die Schraubkappe die Zellkulturflasche ab. Bei manchen Anwendungen ist die Belüftung dieser Kulturflaschen unzureichend.

Davon ausgehend liegt der Erfindung die Aufgabe zugrunde, ein Schraubdeckelgefäß zu schaffen, bei dem eine hinreichende Belüftung sicherer erreicht wird.

Die Aufgabe wird durch ein Schraubdeckelgefäß mit den Merkmalen von Anspruch 1 gelöst. Vorteilhafte Ausgestaltungen des Kulturgefäßes sind in Unteransprüchen angegeben.

Das erfindungsgemäße Schraubdeckelgefäß für den Laboreinsatz hat
- einen eine Kammer umgrenzenden Gefäßkörper,
- einen Rohrabschnitt des Gefäßkörpers, der hinten mit der Kammer verbunden ist und vorn eine Gefäßöffnung aufweist,
- ein Außengewinde auf dem Außenumfang des Rohrabschnittes,
- eine umlaufende erste Dichtfläche am Rohrabschnitt,
- mindestens einen ersten Vorsprung, der an einer Umfangsposition des Rohrabschnittes vom Gefäßkörper vorsteht,
- eine Schraubkappe,
- ein Innengewinde am Innenumfang der Schraubkappe, das mit Gewindespiel auf das Außengewinde aufschraubbar ist,
- eine umlaufende zweite Dichtfläche an der Schraubkappe, die durch Festschrauben der Schraubkappe auf dem Rohrabschnitt abdichtend an die erste Dichtfläche anlegbar ist, und
- mindestens einen zweiten Vorsprung, der in einer Umfangsposition von der Schraubkappe vorsteht,
- wobei der erste Vorsprung in einer kreiszylindrischen Schnittfläche um die Mittelachse des Rohrabschnittes auf der vorderen Seite eine ansteigende Flanke hat und/oder der zweite Vorsprung in einer kreiszylindrischen Schnittfläche um die Mittelachse der Schraubkappe auf der hinteren Seite eine ansteigende Flanke hat, die ersten und zweiten Vorsprünge beim Festschrauben der Schraubkappe auf den Rohrabschnitt zusammentreffen und mit der mindestens einen ansteigenden Flanke aufeinander gleiten, wodurch auf die Schraubkappe eine nach vorn gerichtete Kraft ausgeübt wird, durch die in einer Belüftungsstellung, in der zwischen den ersten und zweiten Dichtflächen ein Belüftungsspalt vorhanden ist, das Innengewinde mit einer Gewindeflanke gegen eine Gewindeflanke des Außengewindes gedrückt wird, die ersten und zweiten Vorsprünge beim weiteren Festschrauben aneinander vorbeibewegt werden und die ersten und zweiten Dichtflächen in eine Dichtstellung gelangen, in der sie abdichtend aneinander anliegen.

In der vorliegenden Anmeldung beziehen sich die Angaben "vorn" und "hinten" auf ein Schraubdeckelgefäß, das mit der Schraubkappe dem Betrachter am nächsten ist, sodass aus der Sicht des Betrachters der Kappenboden vom und das Innengewinde weiter hinten und die Gefäßöffnung vorn und das Außengewinde weiter hinten angeordnet sind.

Der Erfindung liegt die Erkenntnis zugrunde, dass bei der bekannten Zellkulturflasche die Verrastung in der Belüftungsstellung nur in Umfangsrichtung des Flaschenhalses präzise ist. Die Rastung kommt hierbei in Richtung der Rotation des Deckels zustande. Dagegen ist in axialer Richtung des Flaschenhalses durch das Gewindespiel auch in der Belüftungsstellung eine Verlagerung der Schraubkappe auf dem Flaschenhals möglich. Durch diese Verlagerung kann die Dichtung der Schraubkappe unterschiedliche Positionen bezüglich der Flaschenöffnung einnehmen, sodass der Belüftungsquerschnitt variiert. Es ist sogar möglich, dass bei einem Andrücken der Schraubkappe die Dichtung teilweise abdichtet. Infolgedessen ist der Belüftungsquerschnitt nicht genau reproduzierbar und kann der Belüftungsquerschnitt unzureichend sein. Ein weiterer Nachteil ist die schlechte Entformbarkeit der Nuten bei der Herstellung der Schraubkappe durch Spritzgießen. Hierfür wird ein Gewindekern mit entsprechenden Vorsprüngen verwendet, der beim Ausspindeln die Nuten stark deformiert. Hierdurch wird auch die Präzision der Nuten in Umfangsrichtung beeinträchtigt.

Erfindungsgemäß wird in Belüftungsstellung eine präzise Positionierung der Schraubkappe in axialer Richtung durch die ersten und zweiten Vorsprünge erreicht, die sich in der Belüftungsstellung aufeinander abstützen, sodass das Innengewinde mit einer Gewindeflanke gegen eine Gewindeflanke des Außengewindes gepresst wird. Hierdurch wird in der Belüftungsstellung das Gewindespiel aufgehoben. Der Anwender hat durch das fehlende Gewindespiel in axialer Richtung die Sicherheit, dass der Belüftungsspalt zwischen den ersten und zweiten Dichtflächen immer genau reproduziert wird. Somit wird in der Belüftungsstellung stets der konstruktiv vorgegebene Belüftungsquerschnitt erreicht. Eine ausreichende und reproduzierbare Belüftung ist somit sichergestellt. Ausgehend von der Belüftungsstellung ist die Schraubkappe weiter festschraubbar, wobei die ersten und zweiten Vorsprünge aneinander vorbeibewegt werden. Durch weiteres Festschrauben der Schraubkappe wird die Dichtstellung erreicht, in der die ersten und zweiten Dichtflächen abdichtend aneinander anliegen. In der Dichtstellung ist das Schraubdeckelgefäß gas- und flüssigkeitsdicht abgedichtet.

Das Gewindespiel ist erwünscht, um die Schraubkappe zumindest bis zum Zusammentreffen der ersten und weiteren Vorsprünge leichtgängig auf den Rohrabschnitt aufzuschrauben. Zudem stellt sich aufgrund des Gewindespiels in der Belüftungsstellung ein spiralförmig umlaufender Belüftungskanal zwischen Außengewinde und Innengewinde ein, über den der Belüftungsquerschnitt zwischen den Dichtflächen mit der Umgebung verbunden ist. Das axiale Gewindespiel beträgt vorzugsweise 0,5 - 1 mm. Hierdurch ergibt sich ein Belüftungsquerschnitt, der vorzugsweise 6 - 8 mm² beträgt, wobei jedoch auch Querschnitte von bis zu 40 mm²realisiert werden können.

Gemäß einer Ausgestaltung weist der Rohrabschnitt die erste Dichtfläche am Innenumfang des Rohrabschnittes und die Schraubkappe die zweite Dichtfläche an einem vom Kappenboden vorstehenden Stopfen auf. Hierdurch kann ein besonders dichter Verschluss für Flüssigkeiten und Gase erreicht werden. Vorzugsweise ist die erste Dichtfläche am Innenumfang der Gefäßöffnung angeordnet. Es ist aber auch möglich, die erste Dichtfläche in einem Abstand von der Gefäßöffnung im Rohrabschnitt anzuordnen. Ferner kann die erste Dichtfläche an der Stirnseite oder am Außenumfang des Rohrabschnittes vorhanden sein, vorzugsweise zwischen dem Außengewinde und der Stirnfläche. Ferner kann sich die erste Dichtfläche über mehrere der vorgenannten Bereiche erstrecken.

Gemäß einer bevorzugten Ausgestaltung hat der mindestens eine erste Vorsprung in der kreiszylindrischen Schnittfläche um die Mittelachse des Rohrabschnittes auf der vorderen Seite eine abfallende Flanke und/oder hat der mindestens eine zweite Vorsprung in der kreiszylindrischen Schnittfläche um die Mittelachse der Schraubkappe auf der hinteren Seite eine abfallende Flanke, sodass die ersten und zweiten Vorsprünge beim weiteren Festschrauben der Schraubkappe aus der Belüftungsposition in die Dichtposition mit der mindestens einen abfallenden Flanke übereinandergleiten, wodurch die auf die Schraubkappe ausgeübte, nach vorn gerichtete Kraft ganz oder teilweise abgebaut wird, bis die Dichtstellung erreicht wird. Bei dieser Ausgestaltung ist das Gewindespiel wieder vollständig oder teilweise wirksam, wenn die Schraubkappe von der Belüftungsstellung in die Dichtstellung gedreht wird. Dies erleichtert das Festschrauben der Schraubkappe bis zum Erreichen der Dichtstellung. Zudem wird hierdurch eine für die Abdichtung vorteilhafte Zentrierung der ersten Dichtfläche auf die zweite Dichtfläche begünstigt. Schließlich erleichtert diese Ausgestaltung dem Anwender das Erkennen des Erreichens der Belüftungsstellung und der Dichtstellung, weil das Schraubmoment beim Schrauben von der Belüftungsstellung in die Dichtstellung oder umgekehrt spürbar abnimmt und dann wieder ansteigt. Alternativ sind die Vorsprünge so beschaffen, dass das Gewindespiel auf dem gesamten Verstellweg von der Belüftungsstellung bis zur Dichtstellung aufgehoben wird.

Gemäß einer weiteren Ausgestaltung weist der Gefäßkörper mehrere gleichmäßig um den Rohrabschnitt verteilte erste Vorsprünge auf und weist die Schraubkappe eine entsprechende Anzahl gleichmäßig um die Kappenöffnung verteilte zweite Vorsprünge auf. Durch die gleichmäßige Verteilung der ersten und der zweiten Vorsprünge wird eine gleichmäßige Übertragung der Anpresskraff in der Belüftungsstellung und die Einhaltung des gewünschten Belüftungsquerschnittes weiter gefördert.

Gemäß einer bevorzugten Ausgestaltung hat der Gefäßkörper zwei auf diametral einander gegenüberliegenden Seiten des Rohrabschnittes angeordnete erste Vorsprünge und die Schraubkappe zwei auf diametral einander gegenüberliegenden Seiten der Kappenöffnung angeordnete zweite Vorsprünge. Diese Ausgestaltung hat insbesondere fertigungstechnische Vorteile, da die ersten Vorsprünge in der Trennebene eines Spritzgießwerkzeugs angeordnet werden können. Hierdurch wird der Einsatz verhältnismäßig einfacher Spritzgießwerkzeuge begünstigt.

Vorzugsweise weisen der mindestens eine erste Vorsprung und der mindestens eine zweite Vorsprung eine ansteigende Flanke auf. Hierdurch wird ein allmählicher Anstieg der auf die Schraubkappe wirkenden Kraft bis zum Erreichen der Belüftungsstellung begünstigt. Alternativ weist nur der mindestens eine erste Vorsprung oder der mindestens eine zweite Vorsprung eine ansteigende Flanke auf. Vorzugsweise weisen der mindestens eine erste Vorsprung und der mindestens eine zweite Vorsprung eine abfallende Flanke auf. Hierdurch wird ein allmählicher Anstieg der auf die Schraubkappe wirkenden Kraft beim Rückdrehen der Schraubkappe aus der Dichtstellung in die Belüftungsstellung erreicht. Alternativ weist nur der mindestens eine erste Vorsprung oder der mindestens eine zweite Vorsprung eine abfallende Flanke auf.

Gemäß einer weiteren Ausgestaltung ist der mindestens eine erste Vorsprung auf der vorderen Seite nach außen gewölbt und/oder ist der mindestens eine zweite Vorsprung auf der hinteren Seite nach außen gewölbt. Hierdurch wird ein sanfter, feinfühlig aufbringbarer Kraftanstieg bis zum Erreichen der Belüftungsposition begünstigt. Vorzugsweise erstreckt sich die Wölbung des ersten Vorsprungs und/oder des zweiten Vorsprungs jeweils über die ansteigende Flanke und die abfallende Flanke. Alternativ ist nur die ansteigende Flanke und/oder nur die abfallende Flanke mit einer Wölbung nach außen versehen. Gemäß einer anderen Ausgestaltung ist die ansteigende Flanke des ersten Vorsprunges und/oder des zweiten Vorsprunges eine Anschrägung und/oder die abfallende Flanke des ersten Vorsprunges und/oder des zweiten Vorsprungs eine Anschrägung.

Gemäß einer weiteren Ausgestaltung steht der mindestens eine erste Vorsprung hinter dem Außengewinde vom Rohrabschnitt nach außen vor und/oder steht der mindestens eine zweite Vorsprung hinter dem Innengewinde vom Kappenmantel vor. Diese Ausgestaltungen sind vorteilhaft für das Spritzgießen des Kulturgefäßes aus Kunststoff.

Gemäß einer weiteren Ausgestaltung ist der mindestens eine erste Vorsprung ein radial vom Rohrabschnitt vorstehender Zapfen. Gemäß einer bevorzugten Ausgestaltung ist der Zapfen kreiszylindrisch. Diese Ausführung ist materialsparend und in der Herstellung vorteilhaft.

Gemäß einer weiteren Ausgestaltung ist der mindestens eine zweite Vorsprung ein hinter dem Innengewinde vom Kappenmantel vorstehende Höcker. Vorzugsweise übersteigt die Breite des Höckers den Durchmesser des Zapfens, sodass der erste Vorsprung in Belüftungsposition sicher am äußeren Ende des Höckers positioniert ist.

Gemäß einer bevorzugten Ausgestaltung weist der mindestens eine zweite Vorsprung am äußeren Ende eine Rastnut auf, in die der mindestens eine erste Vorsprung in der Belüftungsposition einrastet. Gemäß einer alternativen Ausgestaltung weist der mindestens eine erste Vorsprung am äußeren Ende eine Rastnut auf, in die der mindestens eine zweite Vorsprung in der Belüftungsposition einrastet. Hierdurch wird die Schraubkappe in der Belüftungsposition gesichert. Vorzugsweise ist die Rastnut am äußeren Ende der Wölbung eines Höckers ausgebildet und rastet ein kreiszylindrischer Zapfen in die Rastnut ein. Gemäß einer weiteren Ausgestaltung ist die Rastnut ausgerundet, um das gezielte Herausdrehen des ersten oder zweiten Vorsprunges aus der Rastnut zu erleichtern. Vorzugsweise ist die Rastnut an den beiden seitlichen Übergängen zum äußeren Ende des ersten oder zweiten Vorsprunges und/oder an ihrer Basis ausgerundet. Alternativ sind die beiden Nutenflanken der Rastnut angeschrägt.

Gemäß einer weiteren Ausgestaltung steht der mindestens eine zweite Vorsprung vom Kappenmantel nach innen vor. Diese Ausgestaltung vermeidet Kontakt des Anwenders mit dem ersten und dem zweiten Vorsprung, der beispielsweise zur Beschädigung von Schutzhandschuhen führen kann.

Gemäß einer weiteren Ausgestaltung weist die Schraubkappe am äußeren Rand ihres Kappenmantels eine radiale Aufweitung auf und ist der mindestens eine zweite Vorsprung innerhalb der Aufweitung angeordnet. Die Aufweitung begünstigt eine geschützte Unterbringung der zweiten und ersten Vorsprünge und ein sicheres Greifen und Aufschrauben der Schraubkappe.

Gemäß einer weiteren Ausgestaltung weist das Außengewinde und/oder das Innengewinde mindestens eine Abflachung seines Gewindeprofils auf. Die Abflachung hat fertigungstechnische Vorteile, wenn sie beim Spritzgießen in der Trennebene eines Werkzeuges angeordnet ist. Zudem kann der Gasaustausch auch an der Abflachung vorbei erfolgen. Vorzugsweise ist das Gewindeprofil im Bereich der Abflachung bis auf den kreiszylindrischen Umfang des Rohrabschnittes abgeflacht.

Gemäß einer weiteren Ausgestaltung weist die Schraubkappe auf der Außenseite eine Markierung auf, deren Ausrichtung die Belüftungsstellung und/oder die Dichtstellung indiziert. Bei einer Kulturflasche oder einem anderen Kulturgefäß, das beim Kultivieren eine bestimmte Vorzugsstellung hat, bezieht sich die Ausrichtung der Markierung in der Belüftungsstellung oder in der Dichtstellung vorzugsweise auf die Lage des Kulturgefäßes in der Vorzugsstellung. Beispielsweise ist die Belüftungsstellung durch Ausrichtung der Markierung auf 3-Uhr-Stellung in der Vorzugsstellung indiziert. Gemäß einer weiteren Ausgestaltung ist auf dem Gefäßkörper mindestens eine weitere Markierung angeordnet, auf die die Markierung auf der Schraubkappe in Belüftungsstellung und/oder in Dichtstellung ausgerichtet ist.

Gemäß einer weiteren Ausgestaltung ist das Schraubdeckelgefäß eine Kulturflasche oder ein Röhrchen oder eine Rollerflasche oder eine Spinnerflasche oder ein Erlenmeyerkolben oder eine Laborflasche.

Eine Kulturflasche hat einen flachen, im Wesentlichen rechteckigen Flaschenkörper mit Boden-, Deck- und Seitenwänden, die eine Kammer (Kulturkammer) umgrenzen. Ein Flaschenhals steht von einer schmalen vorderen Seitenwand nach außen vor. Der Flaschenhals weist das Außengewinde auf und ist durch die Schraubkappe verschließbar. Kulturflaschen dienen insbesondere der Vermehrung adhärenter Zellen, die sich an der Oberfläche der Bodenwand anlagern. Die Oberfläche der Bodenwand wird als Wachstumsfläche bezeichnet. Die vier gängigen Flaschengrößen werden allgemein als T-25, T-75, T-175 und T-300 bezeichnet, wobei jede Zahl die jeweils effektive Wachstumsfläche in cm² angibt. Auf dem Markt werden auch andere Formate angeboten mit entweder kleinerer Wachstumsfläche (z.B. 12 cm²) oder einem Vielfachen der obigen Standardflächen (z.B. Multi-Etagen-Flaschen). Kulturflaschen werden auch für die Zellvermehrung von Suspensionszellen verwendet, die keine oder kaum eine Bindung an Oberflächen haben.

Rollerflaschen sind runde Flaschen mit einem Flaschenhals, der mittels einer Schraubkappe verschließbar ist. Sie weisen eine effektive Wachstumsfläche von 700 bis 2.000 cm² auf und eignen sich deshalb besonders für die Massenanzucht adhärenter Zellen. Rollerflaschen werden bei der Kultivierung in einer speziellen Apparatur um ihre Längsachse gedreht.

Spinnerflaschen sind runde Flaschen mit einem großen Volumen mit einem weiten, mittleren Flaschenhals zum Einführen eines Rührers, der von einer Schraubkappe abdichtend am weiten Flaschenhals gehalten ist. Seitlich vom mittleren Flaschenhals ist mindestens ein (vorzugsweise zwei) weniger weiter Flaschenhals angeordnet, der der Zuführung und Entnahme von Medium dient und mittels einer Schraubkappe verschlossen ist. Spinnerflaschen können als Alternative zu Rollerflaschen zur Massenanzucht von Suspensionszellen oder auch von adhärenten Zellen verwendet werden. Im Falle einer Massenanzucht von adhärenten Zellen kann zur Vergrößerung der effektiven Wachstumsfläche ein Trägermaterial in das Anzuchtmedium eingebracht werden, das von einem Rührwerk ständig in Suspension gehalten wird und auf dem die Zellen wachsen können. So können effektive Wachstumsflächen von 700 bis 18.000 cm² pro Gramm Trägermaterial erreicht werden.

Weitere Einzelheiten zur Ausführung und Anwendung von Kulturflaschen, Rollerflaschen und Spinnerflaschen sind beschrieben in "Zell- und Gewebekultur" von Lindl und Gstraunthaler, Spektrum Akademischer Verlag, Heidelberg 2008, 6. Auflage, Seiten 71 bis 72, 323 bis 325, 330 bis 332. Die Erfindung betrifft alle bekannten Ausführungen und Anwendungen von Kulturflaschen, Rollerflaschen und Spinnerflaschen.

Röhrchen und Conical Tubes sind einfache zylindrische und am unteren Ende konische oder kugelförmige Gefäße, die an einem oberen Ende einen Rohrabschnitt mit Außengewinde aufweisen, auf den die Schraubkappe geschraubt ist. Röhrchen können für die Kultivierung von Suspensions- und Spheroidzellen, für die Kultivierung von aeroben Bakterien, Hefen und anderen Mikroorganismen sowie für die Lagerung von Komponenten und Flüssigkeiten eingesetzt werden.

Im Rahmen der Massenanzucht von Zellen werden die genannten Gefäßtypen oder zumindest ein Teil davon sequentiell in der Reihenfolge Röhrchen, Zellkulturflaschen, Rollerflaschen und Spinnerflaschen eingesetzt.

Erlenmeyerkolben können für die Kultivierung von Suspensions- und Spheroidzellen, von adhärenten Zellen und für die Kultivierung von aeroben Bakterien, Hefen und anderen Mikroorganismen eingesetzt werden.

Laborflaschen werden im Labor beispielsweise zur Lagerung von Flüssigkeiten verwendet.

Bei einer Laborflasche oder einem anderen Laborgefäß kann die Schraubkappe vor dem Lösen vom Gefäßkörper zunächst in Belüftungsstellung gebracht werden, um allmählich Dämpfe abzulassen, die schlagartig austreten würden, wenn die Schraubkappe sogleich vom Gefäßkörper gelöst würde. Dies kann insbesondere vorteilhaft sein, wenn die Laborflasche mit einer Flüssigkeit mit hohem Dampfdruck gefüllt ist bzw. mit einer Flüssigkeit, die aggressive oder gesundheitsschädliche Dämpfe freisetzt.

Gemäß einer weiteren Ausgestaltung ist der Gefäßkörper aus Polystyrol oder aus Polycarbonat oder aus einem anderen Kunststoff hergestellt. Diese Materialien sind insbesondere für die Anhaftung von Zellen und im Hinblick auf ihre hohe Durchsichtigkeit vorteilhaft. Die Schraubkappe ist gemäß einer weiteren Ausgestaltung aus Polypropylen oder aus Polyethylen oder aus Polytetrafluorethylen oder aus einem anderen Kunststoff oder aus einer Kombination der genannten Materialien hergestellt.

Die Erfindung wird nachfolgend anhand der anliegenden Zeichnungen von Ausführungsbeispielen näher erläutert. In den Zeichnungen zeigen:
- Fig. 1: eine Kulturflasche mit dem Deckel in Belüftungsposition in Vorderansicht (Fig. 1a), in einer Perspektivansicht schräg von vom und von der linken Seite (Fig. 1b), in Draufsicht (Fig. 1c), in einer teilweisen geschnittenen Seitenansicht von der linken Seite (Fig. 1d) und in einem vergrößerten Detail 1e von Fig. 1d (Fig. 1e);
- Fig. 2: die Kulturflasche mit der Schraubkappe in Dichtstellung in Vorderansicht (Fig. 2a), in Rückansicht (Fig. 2b), in einer teilweise geschnittenen Seitenansicht von der linken Seite (Fig. 2c) und in einem vergrößerten Detail 2d von Fig. 2c (Fig. 2d);
- Fig. 3: den Gefäßkörper derselben Kulturflasche in Seitenansicht von der linken Seite (Fig. 3a), in Draufsicht (Fig. 3b), in Rückansicht (Fig. 3c), einem Längsschnitt (Fig. 3d) und in einem vergrößerten Detail von Fig. 3d (Fig. 3e);
- Fig. 4: die Schraubkappe derselben Kulturflasche in Vorderansicht (Fig. 4a), Draufsicht (Fig. 4b), einen Schnitt entlang der Linie c-c von Fig. 4a (Fig. 4c), Seitenansicht (Fig. 4d) und Rückansicht (Fig. 4e);
- Fig. 5: eine Spinnerflaschen (Fig. 5a), eine Rollerflasche (Fig. 5b), einen Erlenmeyerkolben (Fig. 5c) und ein Röhrchen (Fig. 5d), jeweils in einer Perspektivansicht von oben und von der Seite;
- Fig. 6: die Spinnerflasche (Fig. 6a), die Rollerflasche (Fig. 6b), den Erlenmeyerkolben (Fig. 6c) und das Röhrchen (Fig. 6d), jeweils in einer Seitenansicht;
- Fig. 7: die Gefäßkörper von Spinnerflasche (Fig. 7a), Rollerflasche (Fig. 7b), Erlenmeyerkolben (Fig. 7c) und Röhrchen (Fig. 7d), jeweils in einer Perspektivansicht von unten und von der Seite;
- Fig. 8: die Gefäßkörper von Spinnerflasche (Fig. 8a), Rollerflasche (Fig. 8b), Erlenmeyerkolben (Fig. 8c) und Röhrchen (Fig. 8d), jeweils in einer Seitenansicht.

Nachfolgend beziehen sich die Angaben "oben", "oberhalb", "unten" und "unterhalb" auf die Anordnung einer Kulturflasche mit der Bodenwand auf einem horizontalen Untergrund und der Deckwand oberhalb der Bodenwand.

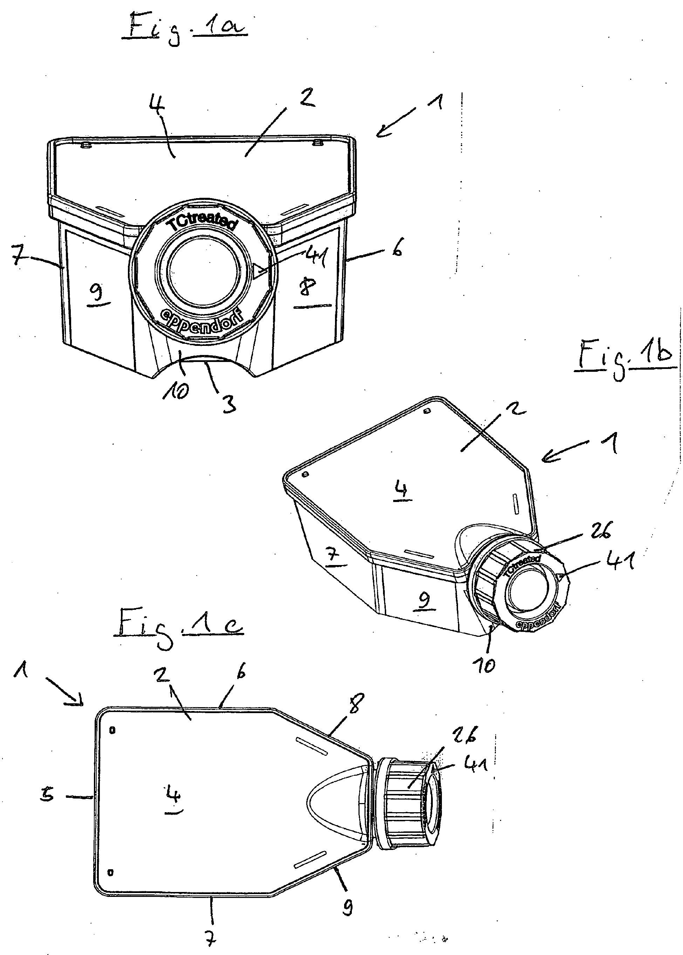

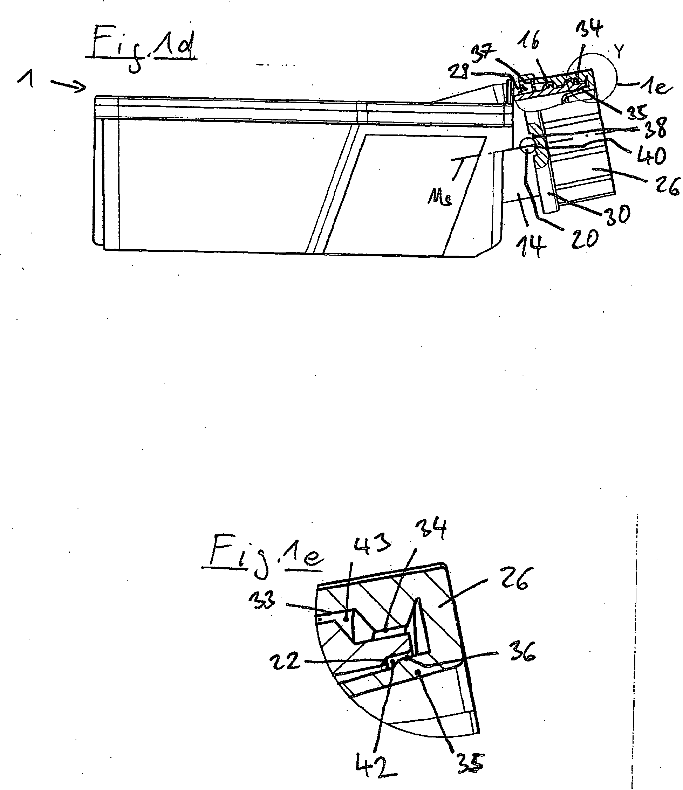

Gemäß Fig. 1 bis 3 hat ein erfindungsgemäßes Kulturgefäß 1 (nachfolgend "Kulturflasche" genannt), einen Gefäßkörper 2 (nachfolgend "Flaschenkörper" genannt) mit einer im Wesentlichen ebenen Bodenwand 3 und einer im Wesentlichen ebenen Deckwand 4, die parallel zueinander ausgerichtet sind. Die Bodenwand 3 und die Deckwand 4 haben jeweils hinten einen im Wesentlichen rechteckigen Abschnitt und vorn einen im Wesentlichen trapezförmigen Abschnitt.

Der Abstandsbereich zwischen der Bodenwand 3 und der Deckwand 4 ist durch Seitenwände 5, 6, 7, 8, 9, 10 überbrückt, die mit den Rändern der Bodenwand 3 und der Deckwand 4 verbunden sind.

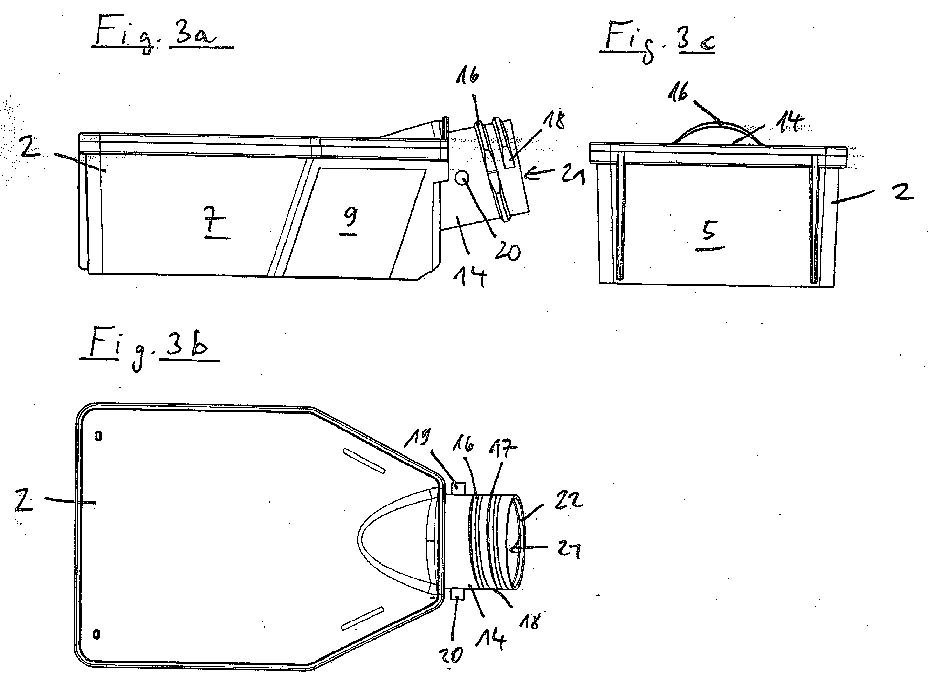

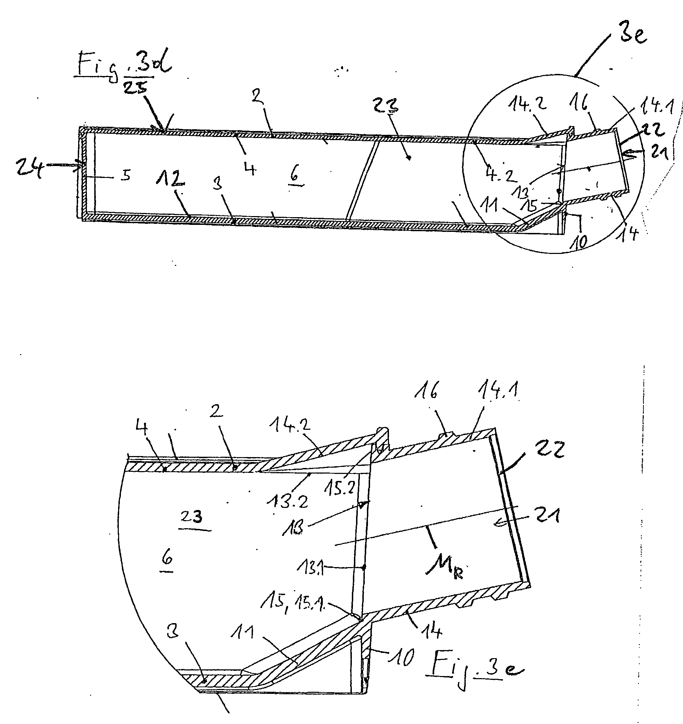

Der vordere Rand des trapezförmigen Abschnitts der Bodenwand ist mit einer trapezförmigen Schrägwand 11 verbunden. Der vordere Rand der Schrägwand 11 endet ein kleines Stück hinter dem vorderen Rand der Deckwand 4. Zwischen diesem Rand der Schrägwand 11 und dem vorderen Rand der Deckwand 4 ist die vordere Seitenwand 10 angeordnet. Die vordere Seitenwand 10 erstreckt sich unterhalb des vorderen Randes der Schrägwand 11 bis auf das Niveau der Unterseite der Bodenwand 3 (vgl. Fig. 3d und 3e).

Die vorderen längsseitigen Seitenwände 8, 9 überbrücken auch den Abstand zwischen längsseitigen Rändern der Schrägwand 11 und den vorderen längsseitigen Rändern der Deckwand 4.

Die Oberseite der Bodenwand ist eine ebene Wachstumsfläche 12.

In einem an die Deckwand 4 angrenzenden Bereich der vorderen Seitenwand 10 und in dem angrenzenden Bereich der Deckwand 4 ist eine Öffnung 13 vorhanden. Ein unterer Öffnungsabschnitt 13.1 der Öffnung 13, der in der vorderen Seitenwand 10 angeordnet ist, ist oberhalb der Schrägwand 11 angeordnet. Die Öffnung 13 ist somit in vertikaler Richtung von der Bodenwand 3 beabstandet.

Ein oberer Öffnungsabschnitt 13.2, der in der Deckwand 4 angeordnet ist, erweitert sich zur vorderen Seitenwand 10 hin.

Ein hohlzylindrischer Rohrabschnitt 14 (nachfolgend "Flaschenhals" genannt) ist mit dem Rand der Öffnung 13 fest verbunden. Der Flaschenhals 14 ist mit seiner Mittelachse M_{R} spitzwinklig zur Bodenwand 3 geneigt. Am vorderen Ende steht der Flaschenhals 14 nach oben über den Abschnitt der Deckwand 4 hinaus, der parallel zur Bodenwand 3 ausgerichtet ist.

Der Flaschenhals 14 hat einen kreisförmigen Querschnitt. Er weist einen ersten Flaschenhalsteil 14.1 auf, der die Form eines Hohlzylinders hat. Der erste Flaschenhalsteil 14.1 ist an einem unteren Randabschnitt 15.1 eines stirnseitigen Randes 15 fest mit dem Rand des unteren Öffnungsabschnitts 13.1 verbunden (vgl. Fig. 3d und 3e).

Ferner weist der Flaschenhals 14 einen zweiten Flaschenhalsteil 14.2 auf, der ein Hohlzylindersegment ist. Der zweite Flaschenhalsteil 14.2 ist an den seitlichen Rändern mit dem ellipsenförmigen Rand des oberen Öffnungsabschnitts 13.2 verbunden. An seinem stirnseitigen Rand ist er mit dem oberen Randabschnitt 15.2 des ersten Flaschenhalsteils 14.1 verbunden, der nach oben über die vordere Seitenwand 10 hinaussteht.

Somit durchdringt der Flaschenhals 14 sowohl den oberen Bereich der vorderen Seitenwand 10 als auch den vorderen Bereich der Deckwand 4. Dies ist im Hinblick auf die Entnahme von Zellen von der Wachstumsfläche 12 mit Hilfe einer Pipette oder eines Schabers besonders vorteilhaft. Die Erfindung ist nicht auf Kulturflaschen mit dieser vorteilhaften Besonderheit eingeschränkt, sondern kann auch bei Kulturflaschen zur Anwendung kommen, bei denen der Flaschenhals nur eine vordere Seiten wand 10 und keine Deckwand 4 durchdringt.

Der Flaschenhals 14 weist außen am Umfang ein Außengewinde 16 für die Verschraubung einer Schraubkappe auf. Das Außengewinde 16 hat vorzugsweise einen einzigen Gewindegang. Dieser läuft beispielsweise zwei Mal um den Umfang des Flaschenhalses 14 um. Das Gewinde ist beispielsweise ein Trapezgewinde, Spitzgewinde öder Flachgewinde. Vorzugsweise hat das Gewindeprofil im Längsschnitt geradlinige Gewindeflanken (vgl. Fig. 3a und 3d).

An den beiden Seiten hat das Außengewinde 16 vertikale Abflachungen 17, 18 des Gewindeprofils.

Auf diametral einander gegenüberliegenden Seiten des Flaschenhalses sitzen nach außen vorspringende erste Vorsprünge in Form von kreiszylindrischen Zapfen 19, 20. Die Zapfen 19, 20 sind auf der vom äußeren Ende des Flaschenhalses 14 abgewandten Seite des Außengewindes 16 auf dem Flaschenhals 14 angeordnet. Sie sind horizontal ausgerichtet und genau auf der Mittelebene des Flaschenhalses 14 positioniert. Aufgrund Ihrer kreiszylindrischen Kontur sind die ersten Vorsprünge auf der Seite des Außengewindes 16 nach außen gewölbt. In einer zylindrischen Kreisfläche um die Mittelachse des Flaschenhalses 14 haben die Zapfen 19, 20 eine zum Außengewinde 16 hin konvergierende Kontur.

Der Flaschenhals 14 hat am vorderen Ende eine Gefäßöffnung 21. Ferner hat er hat am vorderen Ende an seinem Innenumfang eine umlaufende, konische erste Dichtfläche 22. Die erste Dichtfläche 22 umgibt die Gefäßöffnung 21.

Die Wände 3 bis 11 begrenzen eine Kammer 23 (auch "Kulturkammer 23" genannt).

Die Bodenwand 3 sowie die Seitenwände 5 bis 10 und das erste Flaschenhalsteil 14.1 gehören zu einem Flaschenunterteil 24. Die Deckwand 4 und der zweite Flaschenhalsteil 14.2 gehören zu einem Flaschenoberteil 25.

Das Flaschenoberteil ist am Rand umlaufend mit den oberen Rändern der Seitenwände 5, 6, 7, 8, 9 und am hinteren Ende des ersten Flaschenhalsteils 14.1 verbunden. Die Verbindung ist eine Ultraschall-Schweißverbindung, eine Infrarot-Schweißverbindung oder eine Klebeverbindung. Hinsichtlich der geeigneten konstruktiven Ausgestaltung der Ränder und der Durchführung der Verbindungen wird Bezug genommen auf die Patentanmeldungen EP 13 000 264.5 und US 61/754,180, deren Inhalt hiermit in diese Anmeldung aufgenommen wird.

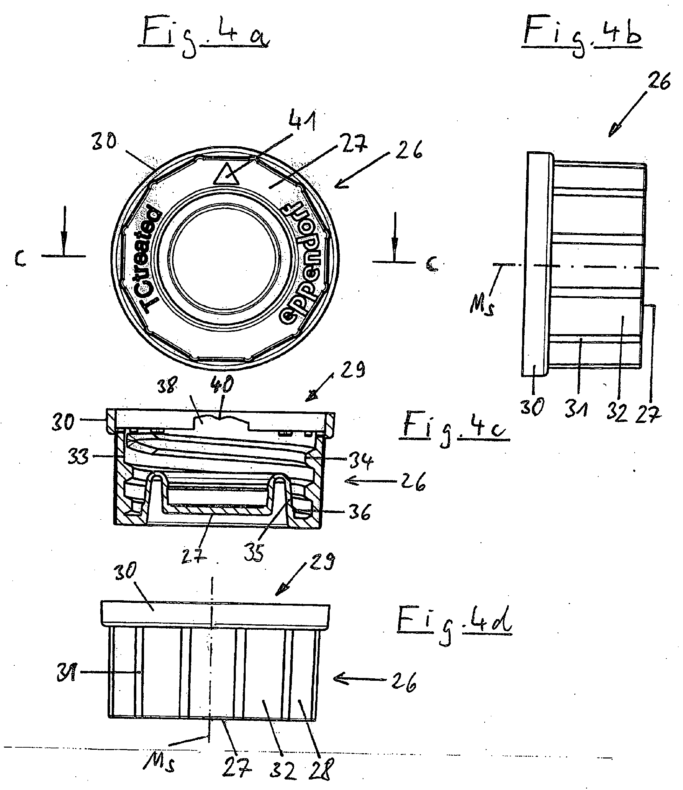

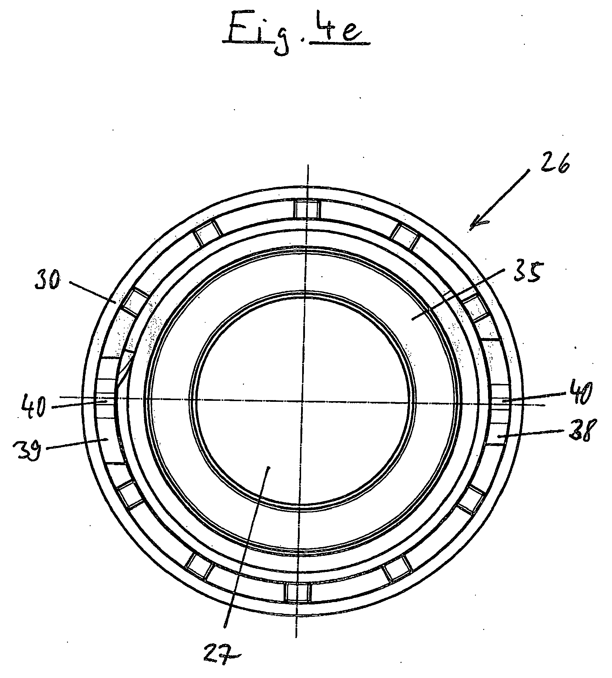

Gemäß Fig. 1d und 4 weist eine Schraubkappe 26 zum Verschließen des Flaschenhalses einen kreisrunden Kappenboden 27 und einen mit dem Rand des Kappenbodens verbundenen, hohlzylindrischen Kappenmantel 28 auf. Gegenüber dem Kappenboden 27 umgrenzt der Kappenmantel 28 eine Kappenöffnung 29. Dort weist der Kappenmantel 28 eine Aufweitung 30 auf. Ausgehend von der Aufweitung 30 sind an der Außenseite des Kappenmantels 29 axial erstreckte Greifrippen 31 angeordnet, die gleichmäßig über den Außenumfang 32 des Kappenmantels 28 verteilt sind.

Am Innenumfang 33 weist die Schraubkappe 26 ein Innengewinde 34 auf. Dieses hat vorzugsweise einen einzigen Gewindegang. Es ist beispielsweise ein Trapezgewinde, Spitzgewinde oder Flachgewinde. Vorzugsweise hat das Gewindeprofil im Längsschnitt geradlinige Gewindeflanken. Im Beispiel läuft das Gewindeprofil zwei Mal um den Innenumfang 33 des Kappenmantels um. Das Innengewinde 34 ist auf das Außengewinde 16 abgestimmt, wobei ein bestimmtes Gewindespiel zwischen Innengewinde und Außengewinde existiert.

Von der Innenseite des Kappenbodens 27 steht nach hinten ein Stopfen 35 in Form eines um die Mittelachse Mₛ der Schraubkappe 26 umlaufenden Wulstes vor. Der Stopfen hat am Außenumfang eine zweite Dichtfläche 36, die ausgebildet ist, an der ersten Dichtfläche 22 des Flaschenhalses 14 abzudichten.

Im Bereich der Aufweitung 30 hat der Kappenanteil eine im Längsschnitt treppenförmige Stirnfläche 37. Dort ist der Kappenmantel 28 am Innenumfang 33 an diametral einander gegenüberliegenden Umfangspositionen mit zweiten Vorsprüngen 38, 39 versehen.

Die zweiten Vorsprünge 38, 39 stehen von der unteren Stufe der treppenförmigen Stirnfläche 37 in axialer Richtung der Schraubkappe 26 vor. Sie stehen hinten nicht über die Aufweitung 30 hinaus. Die zweiten Vorsprünge 38, 39 sind höckerförmig. In einer um die Mittelachse Mₛ der Schraubkappe 26 herum verlaufenden kreiszylindrischen Schnittfläche haben die zweiten Vorsprünge 38, 39 eine Wölbung, die von dem Innengewinde 34 weg nach hinten gerichtet ist. Die zweiten Vorsprünge 38, 39 haben am äußeren Ende jeweils eine ausgerundete Rastnut 40.

Auf der Außenseite weist der Kappenboden 27 eine Markierung 41 auf.

Der Flaschenkörper 2 ist vorzugsweise aus Polystyrol hergestellt. Das Flaschenunterteil 24 und das Flaschenoberteil 25 sind gesondert spritzgegossen und danach miteinander verbunden. Die Schraubkappe 26 ist vorzugsweise einteilig aus Polypropylen spritzgegossen.

Gemäß Fig. 1 bis 3 ist die Schraubkappe 26 auf den Flaschenhals 14 aufgeschraubt. Aufgrund des Gewindespiels ist die Schraubkappe 26 gegenüber den Flaschenhals 14 leicht kippbar. Beim Festschrauben gleiten die ersten Vorsprünge 19, 20 mit ihren ansteigenden Flanken über die ansteigenden Flanken der zweiten Vorsprünge 38, 39, bis sie in die Rastnuten 40 einrasten. Hierbei wird das Innengewinde 34 mit seiner vorderen Gewindeflanke gegen die hintere Gewindeflanke des Außengewindes 16 gepresst (Fig. 1d). Aufgrund dessen ist die Schraubkappe 26 nicht mehr gegenüber dem Flaschenhals 14 kippbar, sondern in einer definierten Ausrichtung gehalten. In dieser Belüftungsstellung liegt die zweite Dichtfläche 36 noch nicht an der ersten Dichtfläche 22 an, sondern ist dazwischen ein umlaufender Belüftungsspalt 42 mit einem bestimmten Belüftungsquerschnitt ausgebildet (Fig. 1e). Dieser ist über den spiralförmig zwischen der vorderen Flanke des Außengewindes 16 und der hinteren Flanke des Innengewindes 34 umlaufenden Belüftungskanal 43 durch die Kappenöffnung 29 mit der Umgebung verbunden. Zusätzlich ist der Belüftungsspalt 42 im Bereich der Abflachungen 17,18 des Außengewindes 16 mit der Kappenöffnung 29 und Umgebung verbunden.

Die Belüftungsstellung wird zum Kultivieren von Zellen, Geweben und Mikroorganismen im Inkubator eingestellt. Dem Anwender wird die Belüftungsstellung dadurch angezeigt, dass sich die Markierung 41 in Dreiuhrstellung befindet (Fig. 1a, b).

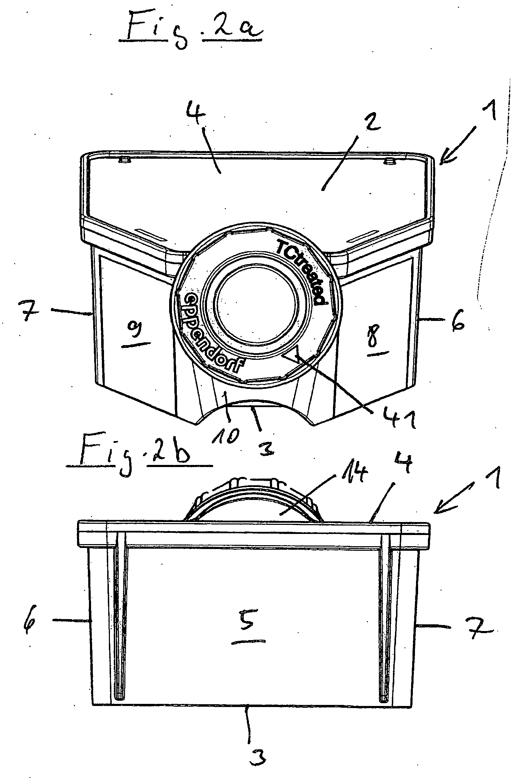

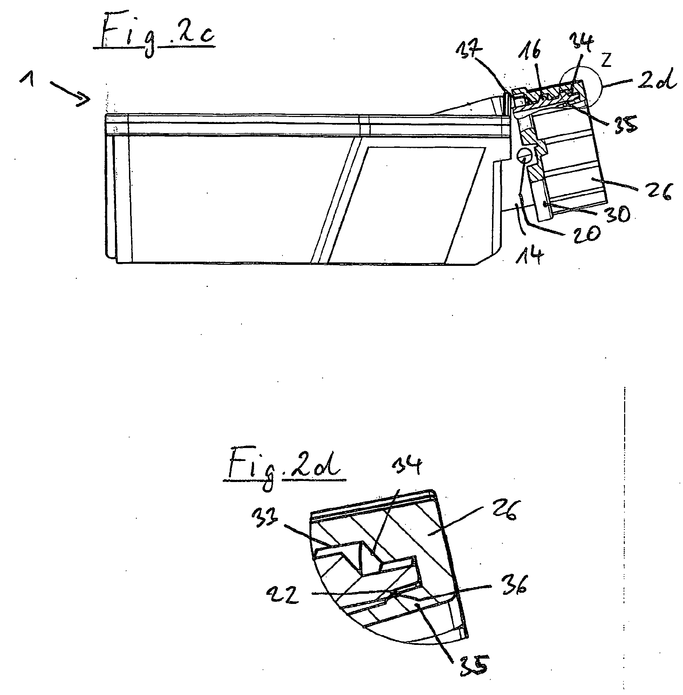

Nach dem Kultivieren kann der Anwender die Schraubkappe 26 durch weiteres Festschrauben der Schraubkappe 26 schließen. Die Dichtstellung ist erreicht, wenn sich die Markierung 41 in Fünfuhrstellung befindet (Fig. 2a). Beim Festschrauben gleiten die abfallenden Flanken der zweiten Vorsprünge 38, 39 über die abfallenden Flanken der ersten Vorsprünge 19, 20, bis die zweiten Vorsprünge 38, 39 von den ersten Vorsprüngen 19, 20 freikommen. Hierdurch wird das Gewindespiel wieder wirksam, bis die zweite Dichtfläche 36 abdichtend an der ersten Dichtfläche 22 anliegt. Aufgrund des Gewindespiels wird eine genaue Ausrichtung der zweiten Dichtfläche 36 auf die erste Dichtfläche 22 und damit eine gute Abdichtung unterstützt (Fig. 2c und 2d).

Zum Öffnen der Kulturflasche 1 schraubt der Anwender die Schraubkappe 26 in entgegengesetzter Richtung über die Belüftungsstellung hinweg, bis sie vom Flaschenhals getrennt ist.

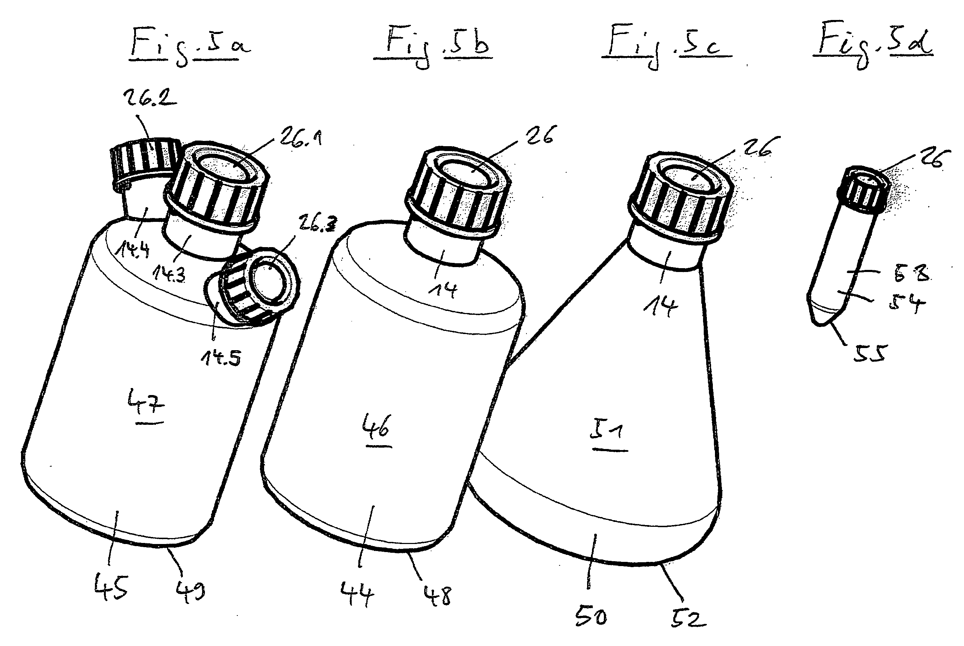

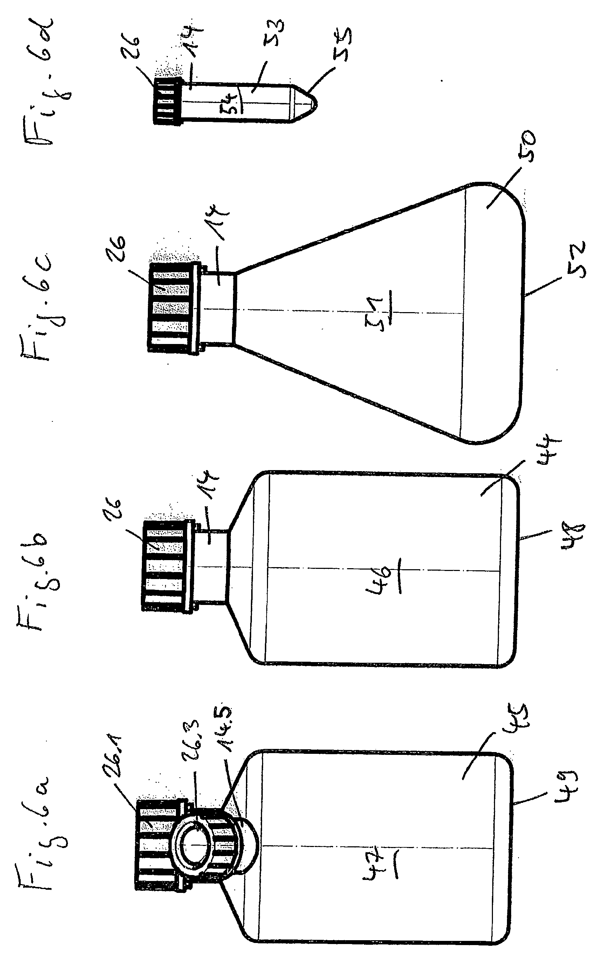

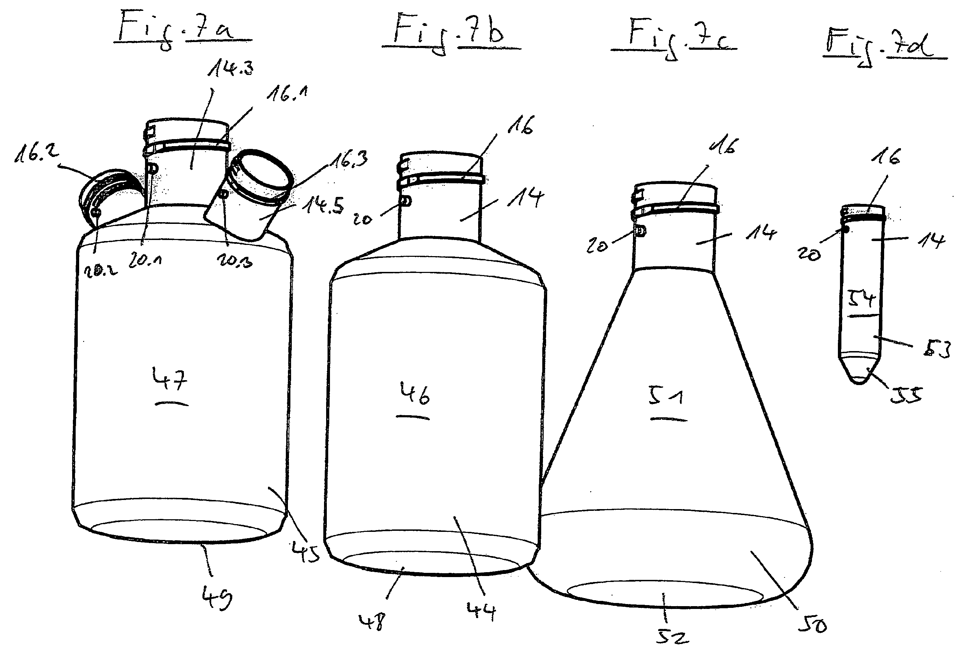

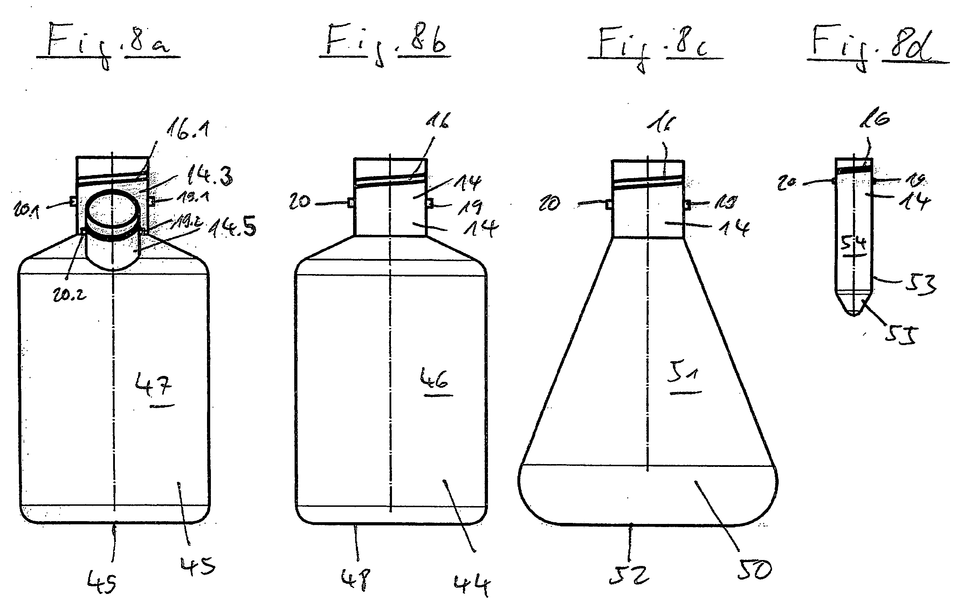

Bei der nachfolgenden Beschreibung der Ausführungsbeispiele von Fig. 5 bis 8 beziehen sich die Angaben "oben" und "unten" auf eine vertikale Ausrichtung der Gefäße mit der Bodenwand unten und der mindestens einen Gefäßöffnung oben.

Eine Rollerflasche 44 und eine Spinnerflasche 45 weisen jeweils einen hohlzylindrischen Gefäßkörper 46, 47 mit einer ebenen Bodenwand 48, 49 und mindestens einem Flaschenhals 14 am oberen Ende auf. Die Rollerflasche 44 hat nur einen einzigen Flaschenhals 14. Die Spinnerflasche 45 hat einen zentralen weiten Flaschenhals 14.3 und seitlich davon in verschiedene Richtungen geneigte, weniger weite Flaschenhälse 14.4, 14.5.

Ein Erlenmeyerkolben 50 hat einen im Wesentlichen konischen Gefäßkörper 51 mit einer ebenen Bodenwand 52 und einem Flaschenhals 14 am oberen Ende. Die Flaschenhälse 14 der vorgenannten Kulturgefäße 44, 45, 50 sind wie der Flaschenhals 14 der Kulturflasche 1 von Fig. 1 bis 3 ausgebildet.

Ein Röhrchen 53 hat einen schlanken, zylindrischen Gefäßkörper 54 mit einer konischen Bodenwand 55 und einem Rohrabschnitt 14 mit Außengewinde 16 am oberen Ende. Der Rohrabschnitt 14 ist entsprechend dem Flaschenhals der oben beschriebenen Kulturflasche 1 ausgebildet.

Die Flaschenhälse 14 bzw. der Rohrabschnitt 14 sind durch Schraubkappen 26 bzw. 26.1, 26.2, 26.3 verschließbar, die entsprechend der Schraubkappe 26 der oben beschriebenen Kulturflasche 1 ausgebildet sind.

Die Kulturgefäße von Fig. 5 bis 8 können durch Einstellen der Schraubkappen 26 bzw. 26.1, 26.2, 26.3 in Belüftungsstellung belüftet und durch Festschrauben der Schraubkappen abgedichtet werden. Sie sind vorteilhaft für die Kultivierung von Proben verwendbar.

### Liste der verwendeten Bezugszeichen

- 1: Kulturgefäß (Kulturflasche)
- 2: Gefäßkörper (Flaschenkörper)
- 3: Bodenwand
- 4: Deckwand
- 4.1: rechteckiger Abschnitt
- 4.2: trapezförmiger Abschnitt
- 5-10: Seitenwände
- 11: Schrägwand
- 12: Wachstumsfläche
- 13: Öffnung
- 13.1: unterer Öffnungsabschnitt
- 13.2: oberer Öffnungsabschnitt
- 14, 14.3-14.5: Rohrabschnitt (Flaschenhals)
- 14.1: erster Flaschenhalsteil
- 14.2: zweiter Flaschenhalsteil
- 15: stirnseitiger Rand
- 15.1: unterer Randabschnitt
- 15.2: oberer Randabschnitt
- 16, 16.1-16.3: Außengewinde
- 17, 18: Abflachung
- 19"19.1-19.3: erste Vorsprünge (Zapfen)
- 20, 20.1-20.3 21: Gefäßöffnung
- 22: erste Dichtfläche
- 23: Kammer (Kulturkammer)
- 24: Flaschenunterteil
- 25: Flaschenoberteil
- 26: Schraubkappe
- 27: Kappenboden
- 28: Kappenmantel
- 29: Kappenöffnung
- 30: Aufweitung
- 31: Greifrippe
- 32: Außenumfang
- 33: Innenumfang
- 34: Innengewinde
- 35: Stopfen
- 36: zweite Dichtfläche
- 37: Stirnfläche
- 38, 39: zweiter Vorsprung
- 40: Rastnut
- 41: Markierung
- 42: Belüftungsspalt
- 43: Belüftungskanal
- 44: Rollerflasche
- 45: Spinnerflasche
- 46,47: Gefäßkörper
- 48,49: Bodenwand
- 50: Erlenmeyerkolben
- 51: Gefäßkörper
- 52: Bodenwand
- 53: Röhrchen
- 54: Gefäßkörper
- 55: Bodenwand

## Patentansprüche

1. Schraubdeckelgefäß für den Laboreinsatz mit
• einem eine Kammer (23),umgrenzenden Gefäßkörper (2),
• einen Rohrabschnitt (14) des Gefäßkörpers (2), der hinten mit der Kammer (23) verbunden ist und vorn eine Gefäßöffnung (21) aufweist,
• einem Außengewinde (16) auf dem Außenumfang des Rohrabschnittes (14),
• einer umlaufenden ersten Dichtfläche (22) am Rohrabschnitt (14),
• mindestens einem ersten Vorsprung (19, 20), der an einer Umfangsposition des Rohrabschnittes (14) vom Gefäßkörper (2) vorsteht,
• einer Schraubkappe (26),
• einem Innengewinde (34) am Innenumfang (33) der Schraubkappe (26), das mit Gewindespiel auf das Außengewinde (16) aufschraubbar ist,
• einer umlaufenden zweiten Dichtfläche (36) an der Schraubkappe (26), die durch Festschrauben der Schraubkappe (26) auf dem Rohrabschnitt (14) abdichtend an die erste Dichtfläche (22) anlegbar ist, und
• mindestens einem zweiten Vorsprung (38, 39), der in einer Umfangsposition der Schraubkappe (26)vorsteht,
• wobei der erste Vorsprung (19, 20) in einer kreiszylindrischen Schnittfläche um die Mittelachse des Rohrabschnittes (14) auf der vorderen Seite eine ansteigende Flanke hat und/oder der zweite Vorsprung (38, 39) in einer kreiszylindrischen Schnittfläche um die Mittelachse der Schraubkappe (26) auf der hinteren Seite eine ansteigende Flanke hat, die ersten und zweiten Vorsprünge (19, 20; 38, 39) beim Festschrauben der Schraubkappe (26) auf dem Rohrabschnitt (14) zusammentreffen und mit der mindestens einen ansteigenden Flanke aufeinander gleiten, wodurch auf die Schraubkappe (26) eine nach vorn gerichtete Kraft ausgeübt wird, durch die in einer Belüftungsstellung, in der zwischen den ersten und zweiten Dichtflächen (22, 36) ein Belüftungsspalt (42) vorhanden ist, das Innengewinde (34) mit einer Flanke gegen eine Gewindeflanke des Außengewindes (16) gedrückt wird, beim weiteren Festschrauben die ersten und zweiten Vorsprünge aneinander vorbeibewegt werden und die ersten und zweiten Dichtflächen (22, 36) in eine Dichtstellung gelangen, in der sie abdichtend aneinander anliegen.

2. Schraubdeckelgefäß nach Anspruch 1, bei dem der Rohrabschnitt (14) die erste Dichtfläche (22) am Innenumfang des Rohrabschnitts (14) und die Schraubkappe (26) die zweite Dichtfläche (36) an einem vom Kappenboden (27) vorstehenden Stopfen (35) aufweist.

3. Schraubdeckelgefäß nach Anspruch 1 oder 2, bei dem der mindestens eine erste Vorsprung (19, 20) in der kreiszylindrischen Schnittfläche um die Mittelachse des Rohrabschnittes (14) auf der vorderen Seite eine abfallende Flanke hat und/oder der mindestens eine zweite Vorsprung (38, 39) in einer kreiszylindrischen Schnittfläche um die Mittelachse der Schraubkappe (26) auf der hinteren Seite eine abfallende Flanke hat, sodass die ersten und zweiten Vorsprünge beim weiteren Festschrauben der Schraubkappe (26) aus der Belüftungsposition in die Dichtposition mit der mindestens einen abfallenden Flanke übereinander gleiten, wodurch die auf die Schraubkappe (26) ausgeübte, nach vorn gerichtete Kraft ganz oder teilweise abgebaut wird, bis die Dichtstellung erreicht wird.

4. Schraubdeckelgefäß nach einem der Ansprüche 1 bis 3, bei dem der Gefäßkörper (2) mehrere gleichmäßig um den Rohrabschnitt verteilte erste Vorsprünge (19, 20) aufweist und die Schraubkappe (26) eine entsprechende Anzahl gleichmäßig um die Kappenöffnung (29) verteilte zweite Vorsprünge (38, 39) aufweist.

5. Schraubdeckelgefäß nach Anspruch 4, bei dem der Gefäßkörper (2) zwei auf diametral einander gegenüberliegenden Seiten des Rohrabschnittes (14) angeordnete erste Vorsprünge (19, 20) und die Schraubkappe (26) zwei auf diametral einander gegenüberliegenden Seiten der Kappenöffnung (29) angeordnete zweite Vorsprünge (38, 39) aufweist.

6. Schraubdeckelgefäß nach einem der Ansprüche 1 bis 5, bei dem der mindestens eine erste Vorsprung (19, 20) auf der vorderen Seite nach außen gewölbt ist und/oder bei dem der mindestens eine zweite Vorsprung (38, 39) auf der hinteren Seite nach außen gewölbt ist.

7. Schraubdeckelgefäß nach einem der Ansprüche 1 bis 6, bei dem der mindestens eine erste Vorsprung (19, 20) hinter dem Außengewinde (16) vom Rohrabschnitt (14) nach außen vorsteht und/oder bei dem der mindestens eine zweite Vorsprung hinter dem Innengewinde (34) vom Kappenmantel (28) vorsteht.

8. Schraubdeckelgefäß nach einem der Ansprüche 1 bis 7, bei dem der mindestens eine erste Vorsprung (19, 20) ein hinter dem Außengewinde (16) radial vom Rohrabschnitt (14) vorstehender kreiszylindrischer Zapfen ist und/oder bei dem der mindestens eine zweite Vorsprung (38, 39) ein hinter dem Innengewinde (34) vom Kappenmantel vorstehender Höcker ist.

9. Schraubdeckelgefäß nach einem der Ansprüche 1 bis 8, bei dem der mindestens eine zweite Vorsprung (38, 39) am äußeren Ende eine Rastnut (40) aufweist, in die der mindestens eine erste Vorsprung (19, 20) in der Belüftungsposition einrastet oder bei dem der mindestens eine erste Vorsprung (19, 20) am äußeren Ende eine Rastnut (40) aufweist, in die der mindestens eine zweite Vorsprung (38, 39) in der Belüftungsposition einrastet.

10. Schraubdeckelgefäß nach Anspruch 9, bei dem die Rastnut (40) ausgerundet ist.

11. Schraubdeckelgefäß nach einem der Ansprüche 1 bis 10, bei dem der mindestens eine zweite Vorsprung (38, 39) vom Kappenmantel (28) nach innen vorsteht.

12. Schraubdeckelgefäß nach einem der Ansprüche 1 bis 11, bei dem die Schraubkappe (26) am äußeren Rand ihres Kappenmantels (28) eine radiale Aufweitung (30) aufweist und der mindestens eine zweite Vorsprung (38, 39) innerhalb der Aufweitung (30) angeordnet ist.

13. Schraubdeckelgefäß nach einem der Ansprüche 1 bis 12, bei dem das Außengewinde (16) und/oder das Innengewinde (34) mindestens eine Abflachung (17, 18) des Gewindeprofils aufweist.

14. Schraubdeckelgefäß nach einem der Ansprüche 1 bis 13, bei dem die Schraubkappe (26) auf der Außenseite eine Markierung (41) aufweist.

15. Schraubdeckelgefäß nach einem der Ansprüche 1 bis 14, das eine Kulturflasche (1) oder ein Röhrchen (53) oder eine Rollerflasche (44) oder eine Spinnerflasche (45) oder ein Erlenmeyerkolben (50) oder eine Laborflasche ist.

16. Schraubdeckelgefäß nach einem der Ansprüche 1 bis 15, dessen Gefäßkörper (2) aus Polystyrol oder aus Polycarbonat oder aus einem anderen Kunststoff hergestellt ist und/oder dessen Schraubkappe (26) aus Polypropylen oder aus Polyethylen oder aus Polytetrafluorethylen oder aus einem anderen Kunststoff oder aus einer Kombination der genannten Materialien hergestellt ist.

## Claims

1. A screw cap lidded container for laboratory use comprising
• a container body (2) defining a chamber (23),
• a tubular section (14) of the container body (2) that is connected at the rear to the chamber (23) and has a container opening (21) in the front,
• an external thread (16) on the outer periphery of the tubular section (14),
• a circumferential first sealing surface (22) on the tubular section (14),
• at least one first projection (19, 20), which projects from the container body (2) at a peripheral position of the tubular section (14),
• a screw cap (26),
• an internal thread (34) on the inner periphery (33) of the screw cap (26) that can be screwed onto the external thread (16) with thread play,
• a circumferential second sealing surface (36) on the screw cap (26), that by screwing the screw cap (26) tightly onto the tubular section (14) can be brought into sealing contact with the first sealing surface (22), and
• at least one second projection (38, 39), which projects from the screw cap (26) at a peripheral position of the screw cap (26),
• wherein the first projection (19, 20) on the front side, in a circular cylindrical cut surface about the central axis of the tubular section (14), has an increasing shoulder, and/or the second projection (38, 39) on the back side, in a circular cylindrical cut surface about the central axis of the screw cap (26), has an increasing shoulder, wherein when screwing the screw cap (26) tightly onto the tubular section (14), the first and second projections (19, 20; 38, 39) come into contact and glide onto each other with the at least one increasing shoulder, whereby a forward directed force is exerted on the screw cap (26), through which, in a ventilation setting in which a ventilation gap (42) is present between the first and second sealing surfaces (22, 36), the internal thread (34) is pressed with a flank against a thread flank of the external thread (16), the first and second projections are moved past each other during further screwing tightly and the first and second sealing surfaces (22, 36) attain a sealed setting in which they lie sealed against each other.

2. The screw cap lidded container according to claim 1, in which the tubular section (14) has the first sealing surface (22) on the inner periphery of the tubular section (14) and the screw cap (26) has the second sealing surface (36) on a stopper (35) projecting from the cap bottom (27).

3. The screw cap lidded container according to claim 1 or 2, in which the at least one first projection (19, 20) on the front side, in the circular cylindrical cut surface about the central axis of the tubular section (14), has a decreasing shoulder, and/or the at least one second projection (38, 39) on the back side, in a circular cylindrical cut surface about the central axis of the screw cap (26), has a decreasing shoulder, such that during further screwing the screw cap (26) tightly from the ventilation position into the sealed position, the first and second projections glide over each other with the at least one decreasing shoulder, whereby the forward directed force exerted on the screw cap (26) is completely or partially absorbed until the sealed setting is attained.

4. The screw cap lidded container according to one of the claims 1 to 3, in which the container body (2) has a plurality of first projections (19, 20) distributed uniformly about the tubular section, and the screw cap (26) has a corresponding number of second projections (38, 39) distributed uniformly about the cap opening (29).

5. The screw cap lidded container according to claim 4, in which the container body (2) has two first projections (19, 20) arranged on diametrically opposed sides of the tubular section (14), and the screw cap (26) has two second projections (38, 39) arranged on diametrically opposed sides of the cap opening (29).

6. The screw cap lidded container according to one of the claims 1 to 5, in which the at least one first projection (19, 20) is convex on the front side, and/or in which the at least one second projection (38, 39) is convex on the back side.

7. The screw cap lidded container according to one of the claims 1 to 6, in which the at least one first projection (19, 20) projects outward from the tubular section (14) behind the external thread (16), and/or in which the at least one second projection projects from the cap casing (28) behind the internal thread (34).

8. The screw cap lidded container according to one of the claims 1 to 7, in which the at least one first projection (19, 20) is a circular cylindrical pin projecting radially from the tubular section (14) behind the external thread (16), and/or in which the at least one second projection (38, 39) is a hump projecting from the cap casing behind the internal thread (34).

9. The screw cap lidded container according to one of the claims 1 to 8, in which the at least one second projection (38, 39) on an outer end has a latching groove (40) into which the at least one first projection (19, 20) latches in the ventilation position, or in which the at least one first projection (19, 20) on the outer end has a latching groove (40) into which the at least one second projection (38, 39) latches in the ventilation position.

10. The screw cap lidded container according to claim 9, in which the latching groove (40) is rounded.

11. The screw cap lidded container according to one of the claims 1 to 10, in which the at least one second projection (38, 39) projects inward from the cap casing (28).

12. The screw cap lidded container according to one of the claims 1 to 11, in which the screw cap (26) has a radial widening (30) on the outer edge of the cap casing (28) thereof, and the at least one second projection (38, 39) is arranged within the widening (30).

13. The screw cap lidded container according to one of the claims 1 to 12, in which the external thread (16) and/or the internal thread (34) have at least one flattening (17, 18) of the thread profile.

14. The screw cap lidded container according to one of the claims 1 to 13, in which the screw cap (26) has a marking (41) on the outside.

15. The screw cap lidded container according to one of the claims 1 to 14, that is a culture flask (1), or a test tube (53), or a roller bottle (44), or a spinner bottle (45), or an Erlenmeyer flask (50), or a laboratory bottle.

16. The screw cap lidded container according to one of the claims 1 to 15, whose container body (2) is produced from polystyrene or polycarbonate or from another plastic and/or whose screw cap (26) is produced from polypropylene or polyethylene or polytetrafluoroethylene or from another plastic or from a combination of the named materials.

## Revendications

1. Récipient à couvercle à visser pour un usage en laboratoire, comprenant :
• un corps de récipient (2) entourant une chambre (23),
• une section de tuyau (14) du corps de récipient (2), laquelle est reliée à la chambre (23) à l'arrière et présente une ouverture de récipient (21) à l'avant,
• un filetage extérieur (16) sur le pourtour extérieur de la section de tuyau (14),
• une première surface d'étanchéité périphérique (22) sur la section de tuyau (14),
• au moins une première saillie (19, 20) faisant saillie à partir du corps de récipient (2) en une position périphérique de la section de tuyau (14),
• un capuchon fileté (26),
• un filetage intérieur (34) sur le pourtour intérieur (33) du capuchon fileté (26), lequel peut être vissé avec un jeu de filetages sur le filetage extérieur (16),
• une deuxième surface d'étanchéité périphérique (36) sur le capuchon fileté (26), laquelle peut être appliquée de façon étanche sur la première surface d'étanchéité (22) par vissage du capuchon fileté (26) sur la section de tuyau (14), et
• au moins une deuxième saillie (38, 39) faisant saillie en une position périphérique du capuchon fileté (26),
• la première saillie (19, 20) présentant un flanc ascendant sur le côté avant, dans une surface de coupe cylindrique circulaire autour de l'axe médian de la section de tuyau (14), et/ou la deuxième saillie (38, 39) présentant un flanc ascendant sur le côté arrière, dans une surface de coupe cylindrique circulaire autour de l'axe médian du capuchon fileté (26), les première et deuxième saillies (19, 20 ; 38, 39) se rejoignant lors du vissage du capuchon fileté (26) sur la section de tuyau (14) et glissant l'une sur l'autre avec l'au moins un flanc ascendant, exerçant ainsi une force orientée vers l'avant et exercée sur le capuchon fileté (26), par laquelle le filetage intérieur (34) est pressé avec un flanc contre un flanc de filetage du filetage extérieur (16), dans une position d'aération dans laquelle une fente d'aération (42) est présente entre les première et deuxième surfaces d'étanchéité (22, 36), les première et deuxième saillies sont déplacées l'une au devant l'autre lors d'un vissage supplémentaire, et les première et deuxième surfaces d'étanchéité (22, 36) parviennent dans une position d'étanchéité dans laquelle elles s'appliquent l'une sur l'autre de façon étanche.

2. Récipient à couvercle à visser selon la revendication 1, dans lequel la section de tuyau (14) présente la première surface d'étanchéité (22) sur le pourtour intérieur de la section de tuyau (14), et le capuchon fileté (26) présente la deuxième surface d'étanchéité (36) sur un bouchon (35) faisant saillie à partir du fond du capuchon (27).

3. Récipient à couvercle à visser selon la revendication 1 ou 2, dans lequel l'au moins une première saillie (19, 20) présente un flanc descendant sur le côté avant, dans une surface de coupe cylindrique circulaire autour de l'axe médian de la section de tuyau (14), et/ou l'au moins une deuxième saillie (38, 39) présente un flanc descendant sur le côté carrière, dans une surface de coupe cylindrique circulaire autour de l'axe médian du capuchon fileté (26), de sorte que les première et deuxième saillies glissent l'une par-dessus l'autre avec l'au moins un flanc descendant, lors d'un vissage supplémentaire du capuchon fileté (26) hors de la position d'aération vers la position d'étanchéité, supprimant ainsi entièrement ou partiellement la force orientée vers l'avant et exercée sur le capuchon fileté (26), jusqu'à ce que la position d'étanchéité soit atteinte.

4. Récipient à couvercle à visser selon l'une des revendications 1 à 3, dans lequel le corps de récipient (2) présente plusieurs premières saillies (19, 20) réparties de façon régulière autour de la section de tuyau, et le capuchon fileté (26) présente un nombre correspondant de deuxièmes saillies (38, 39) réparties de façon régulière autour de l'ouverture de capuchon (29).

5. Récipient à couvercle à visser selon la revendication 4, dans lequel le corps de récipient (2) présente deux premières saillies (19, 20) agencées sur des côtés diamétralement opposés de la section de tuyau (14), et le capuchon fileté (26) présente deux deuxièmes saillies (38, 39) agencées sur des côtés diamétralement opposés de l'ouverture de capuchon (29).

6. Récipient à couvercle à visser selon l'une des revendications 1 à 5, dans lequel l'au moins une première saillie (19, 20) est voûtée vers l'extérieur sur le côté avant, et/ou dans lequel l'au moins une deuxième saillie (38, 39) est voûtée vers l'extérieur sur le côté arrière.

7. Récipient à couvercle à visser selon l'une des revendications 1 à 6, dans lequel l'au moins une première saillie (19, 20) fait saillie vers l'extérieur à partir de la section de tuyau (14) derrière le filetage extérieur (16), et/ou dans lequel l'au moins une deuxième saillie fait saillie à partir du manteau de capuchon (28) derrière le filetage intérieur (34).

8. Récipient à couvercle à visser selon l'une des revendications 1 à 7, dans lequel l'au moins une première saillie (19, 20) est un téton cylindrique circulaire faisant saillie radialement à partir de la section de tuyau (14) derrière le filetage extérieur (16), et/ou dans lequel l'au moins une deuxième saillie (38, 39) est un bossage faisant saillie à partir du manteau de capuchon derrière le filetage intérieur (34).

9. Récipient à couvercle à visser selon l'une des revendications 1 à 8, dans lequel l'au moins une deuxième saillie (38, 39) présente une rainure d'encliquetage (40) à l'extrémité extérieure, dans laquelle l'au moins une première saillie (19, 20) s'engage dans la position d'aération, ou dans lequel l'au moins une première saillie (19, 20) présente une rainure d'encliquetage (40) à l'extrémité extérieure, dans laquelle l'au moins une deuxième saillie (38, 39) s'engage dans la position d'aération.

10. Récipient à couvercle à visser selon la revendication 9, dans lequel la rainure d'encliquetage (40) est arrondie.

11. Récipient à couvercle à visser selon l'une des revendications 1 à 10, dans lequel l'au moins une deuxième saillie (38, 39) fait saillie vers l'intérieur à partir du manteau de capuchon (28).

12. Récipient à couvercle à visser selon l'une des revendications 1 à 11, dans lequel le capuchon fileté (26) présente un élargissement radial (30) sur le bord extérieur de son manteau de capuchon (28), et l'au moins une deuxième saillie (38, 39) est agencée à l'intérieur de l'élargissement (30).

13. Récipient à couvercle à visser selon l'une des revendications 1 à 12, dans lequel le filetage extérieur (16) et/ou le filetage intérieur (34) présente au moins un aplatissement (17, 18) du profil de filetage.

14. Récipient à couvercle à visser selon l'une des revendications 1 à 13, dans lequel le capuchon fileté (26) présente un marquage (41) sur le côté extérieur.

15. Récipient à couvercle à visser selon l'une des revendications 1 à 14, lequel est un flacon de culture (1) ou un petit tube (53) ou un flacon roulant (44) ou un flacon Spinner (45) ou une fiole Erlenmeyer (50) ou un flacon de laboratoire.

16. Récipient à couvercle à visser selon l'une des revendications 1 à 15, dont le corps de récipient (2) est fabriqué à partir de polystyrène ou de polycarbonate ou d'une autre matière plastique, et/ou dont le capuchon fileté (26) est fabriqué à partir de polypropylène ou de polyéthylène ou de polytétrafluoréthylène ou d'une autre matière plastique ou d'une combinaison des matériaux cités.
